# EUROPÄISCHE PATENTSCHRIFT

(11) **EP 1 259 213 B1**
(45) Veröffentlichungstag und Bekanntmachung des Hinweises auf die Patenterteilung: **02.04.2008**
(21) Anmeldenummer: 01915277.6
(22) Anmeldetag: 19.02.2001
(51) Int. Cl.: A61K 8/49, A61K 8/72, A61Q 5/06, A61Q 5/10, D06M 13/352, D06P 1/642

(54) **VERWENDUNG VON PYRROLIDINONCARBONSÄUREN UND POLYMEREN**
USE OF PYRROLIDINONE CARBOXYLIC ACIDS AND POLYMERS
UTILISATION D'ACIDES PYRROLIDINONE CARBOXYLIQUES ET DE POLYMERES

(30) Priorität: 29.02.2000 DE 10009438
(43) Veröffentlichungstag der Anmeldung: 27.11.2002
(73) Patentinhaber: Henkel Kommanditgesellschaft auf Aktien, 40589 Düsseldorf (DE)
(72) Erfinder: MUNK, Gabriele, 22763 Hamburg (DE); POPPE, Elisabeth, 40219 Düsseldorf (DE); DELOWSKY, Jens, 22844 Norderstedt (DE); KURSAWE, Petra, 22527 Hamburg (DE)
(86) Internationale Anmeldenummer: PCT/EP2001/001819
(87) Internationale Veröffentlichungsnummer: WO 2001/064172

(56) Entgegenhaltungen:
- EP-A- 0 873 745
- DE-A- 4 109 976
- DE-A- 19 533 211
- DE-A- 19 536 423
- DE-A- 19 615 145
- DE-C- 4 430 521

## Beschreibung

Die Erfindung betrifft die Verwendung von Pyrrolidinoncarbonsäuren und bestimmten natürlichen oder synthetischen Polymeren zur Verbesserung der Waschechtheit von Färbungen keratinischer Fasern, entsprechende Zubereitungen sowie Verfahren zum Färben von Fasern.

Menschliches Haar wird heute in vielfältiger Weise mit haarkosmetischen Zubereitungen behandelt. Dazu gehören etwa die Reinigung der Haare mit Shampoos, die Pflege und Regeneration mit Spülungen und Kuren sowie das Bleichen, Färben und Verformen der Haare mit Färbemitteln, Tönungsmitteln, Wellmitteln und Stylingpräparaten. Dabei spielen Mittel zur Veränderung oder Nuancierung der Farbe des Kopfhaares eine herausragende Rolle. Sieht man von den Blondiermitteln, die eine oxidative Aufhellung der Haare durch Abbau der natürlichen Haarfarbstoffe bewirken, ab, so sind im Bereich der Haarfärbung im wesentlichen drei Typen von Haarfärbemitteln von Bedeutung:

Für dauerhafte, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, sogenannte Entwicklerkomponenten und Kupplerkomponenten. Die Entwicklerkomponenten bilden unter dem Einfluß von Oxidationsmitteln oder von Luftsauerstoff untereinander oder unter Kupplung mit einer oder mehreren Kupplerkomponenten die eigentlichen Farbstoffe aus. Die Oxidationsfärbemittel zeichnen sich zwar durch hervorragende, lang anhaltende Färbeergebnisse aus. Für natürlich wirkende Färbungen muß aber üblicherweise eine Mischung aus einer größeren Zahl von Oxidationsfarbstoffvorprodukten eingesetzt werden; in vielen Fällen werden weiterhin direktziehende Farbstoffe zur Nuancierung verwendet. Weisen die im Verlauf der Farbausbildung gebildeten bzw. direkt eingesetzten Farbstoffe deutlich unterschiedliche Echtheiten (z. B. UV-Stabilität. Schweißechtheit. Waschechtheit etc.) auf, so kann es mit der Zeit zu einer erkennbaren und daher unerwünschten Farbverschiebung kommen.

Dieses Phänomen tritt verstärkt auf, wenn die Frisur Haare oder Haarzonen unterschiedlichen Schädigungsgrades aufweist. Ein Beispiel dafür sind lange Haare, bei denen die lange Zeit allen möglichen Umwelteinflüssen ausgesetzten Haarspitzen in der Regel deutlich stärker geschädigt sind als die relativ frisch nachgewachsenen Haarzonen.

Für temporäre Färbungen werden üblicherweise Färbe- oder Tönungsmittel verwendet, die als färbende Komponente sogenannte Direktzieher enthalten. Hierbei handelt es sich um Farbstoffmoleküle, die direkt auf das Haar aufziehen und keinen oxidativen Prozeß zur Ausbildung der Farbe benötigen. Zu diesen Farbstoffen gehört beispielsweise das bereits aus dem Altertum zur Färbung von Körper und Haaren bekannte Henna. Diese Färbungen sind gegen Shampoonieren in der Regel deutlich empfindlicher als die oxidativen Färbungen, so daß dann sehr viel schneller eine vielfach unerwünschte Nuancenverschiebung oder gar eine sichtbare "Entfärbung" eintritt.

Schließlich hat in jüngster Zeit ein neuartiges Färbeverfahren große Beachtung gefunden. Bei diesem Verfahren werden Vorstufen des natürlichen Haarfarbstoffes Melanin auf das Haar aufgebracht; diese bilden dann im Rahmen oxidativer Prozesse im Haar naturanaloge Farbstoffe aus. Ein solches Verfahren mit 5,6-Dihydroxyindolin als Farbstoffvorprodukt wurde in der EP-B1-530 229 beschrieben. Bei, insbesondere mehrfacher, Anwendung von Mitteln mit 5,6-Dihydroxyindolin ist es möglich, Menschen mit ergrauten Haaren die natürliche Haarfarbe wiederzugeben. Die Ausfärbung kann dabei mit Luftsauerstoff als einzigem Oxidationsmittel erfolgen, so daß auf keine weiteren Oxidationsmittel zurückgegriffen werden muß. Bei Personen mit ursprünglich mittelblondem bis braunem Haar kann das Indolin als alleinige Farbstoffvorstufe eingesetzt werden. Für die Anwendung bei Personen mit ursprünglich roter und insbesondere dunkler bis schwarzer Haarfarbe können dagegen befriedigende Ergebnisse häufig nur durch Mitverwendung weiterer Farbstoffkomponenten, insbesondere spezieller Oxidationsfarbstoffvorprodukte, erzielt werden. Auch hier können dann Probleme hinsichtlich der Echtheit der Färbungen auftreten.

Es hat nicht an Anstrengungen gefehlt, die Echtheit von Färbungen keratinischer Fasern zu verbessern. Eine Entwicklungsrichtung ist die Optimierung der Farbstoffe selbst bzw. die Synthese neuer, modifizierter Farbstoffmoleküle. Eine weitere Entwicklungsrichtung ist die Suche nach Zusätzen für die Färbemittel, um die Echtheit der Färbungen zu erhöhen. Eine bekannte Problemlösung ist, dem Färbemittel UV-Filter zuzusetzen. Diese Filtersubstanzen werden beim Färbeprozeß zusammen mit dem Farbstoff auf das Haar aufgebracht, wodurch in vielen Fällen eine deutliche Steigerung der Stabilität der Färbung gegen die Einwirkung von Tages- oder Kunstlicht erzielt wird.

Es wurde nun überraschenderweise gefunden, daß durch den Einsatz einer Wirkstoffkombination aus Pyrrolidinoncarbonsäuren und bestimmten natürlichen oder synthetischen Polymeren die Waschechtheit von Färbungen insbesondere keratinischer Fasern signifikant gesteigert werden kann. Unter Waschechtheit im Sinne der Erfindung ist die Erhaltung der ursprünglichen Färbung hinsichtlich Nuance und/oder Intensität zu verstehen, wenn die keratinische Faser dem wiederholten Einfluß von wäßrigen Mitteln, insbesondere tensidhaltigen Mitteln wie Shampoos, ausgesetzt wird.

Ein erster Gegenstand der vorliegenden Erfindung ist daher die Verwendung eines Wirkstoffkomplexes bestehend aus einer Pyrrolidinoncarbonsäure und/oder eines Pyrrolidinoncarbonsäurederivates [(A)] der Formel (I), in der mindestens einer der Substituenten R¹ bis R³ steht für eine Gruppe -COOR⁴, in der R⁴ Wasserstoff, ein Alkalimetallion, ein Erdalkalimetallion oder ein Ammoniumion ⁺NHR⁵R⁶R⁷ ist, in dem R⁵ bis R⁷ unabhängig voneinander Wasserstoff, Alkylgruppen mit 1 bis 22 C-Atomen, Hydroxyalkylgruppen mit 1 bis 4 C-Atomen, Alkenylgruppen mit 2 bis 22 C-Atomen, Acylgruppen mit 2 bis 22 C-Atomen oder gegebenenfalls substituierte aromatische Gruppen mit 6 bis 10 C-Atomen sind. und die restlichen Substituenten R¹ bis R³ für Wasserstoff oder Alkylgruppen mit 1 bis 4 C-Atomen stehen, 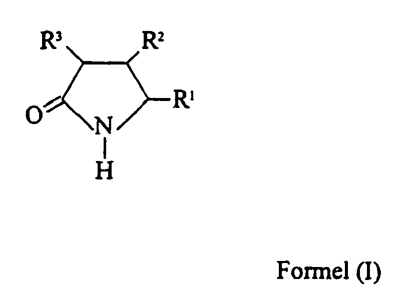 und eines natürlichen oder synthetischen, kationischen oder amphoteren Polymeren (B) zur Verbesserung der Waschechtheit von Färbungen von Fasern, insbesondere von keratinischen Fasern.

Der erfindungsgemäß verwendete Wirkstoffkomplex verbessert die Waschechtheit von Färbungen auf künstlichen Fasern wie Polyestern und natürlichen Fasern wie Baumwolle und insbesondere keratinischen Fasern.

Unter keratinischen Fasern werden erfindungsgemäß Pelze, Wolle, Federn und insbesondere menschliche Haare verstanden.

Die Verbindungen (A) sind Derivate des 2-Pyrrolidinons. Bevorzugte Derivate sind die 2-Pyrrolidinon-3-, -4- und -5-carbonsäure und deren Salze. Bevorzugte Salze dieser Verbindungen sind die Natrium-, Kalium-, Calcium-, Magnesium- und solche Ammoniumsalze, bei denen das Ammoniumion neben Wasserstoff eine bis drei C₁- bis C₄-Alkylgruppen trägt. Besonders bevorzugte Verbindungen (A) sind die 2-Pyrrolidinon-5-carbonsäure und deren Salze. Das Natriumsalz ist ganz besonders bevorzugt.

Die Verbindungen (A) sind in den erfindungsgemäß verwendeten Mitteln bevorzugt in Mengen von 0,05 bis 10 Gew.-%, bezogen auf das gesamte Mittel, enthalten. Mengen von 0,1 bis 5. insbesondere 0,1 bis 3 Gew.%, sind besonders bevorzugt.

Gemäß einer ersten Ausführungsform handelt es sich bei dem Polymer (B) des erfindungsgemäßen Wirkstoffkomplexes um ein kationisches Polymer. Unter kationischen Polymeren sind Polymere zu verstehen, welche in der Haupt- und/oder Seitenkette eine Gruppe aufweisen, welche "temporär" oder "permanent" kationisch sein kann. Als "permanent kationisch" werden erfindungsgemäß solche Polymere bezeichnet, die unabhängig vom pH-Wert des Mittels eine kationische Gruppe aufweisen. Dies sind in der Regel Polymere, die ein quartäres Stickstoffatom, beispielsweise in Form einer Ammoniumgruppe, enthalten. Bevorzugte kationische Gruppen sind quartäre Ammoniumgruppen. Insbesondere solche Polymere, bei denen die quartäre Ammoniumgruppe über eine C₁₋₄-Kohlenwasserstoffgruppe an eine aus Acrylsäure, Methacrylsäure oder deren Derivaten aufgebaute Polymerhauptkette gebunden sind, haben sich als besonders geeignet erwiesen. Homopolymere der allgemeinen Formel (II), 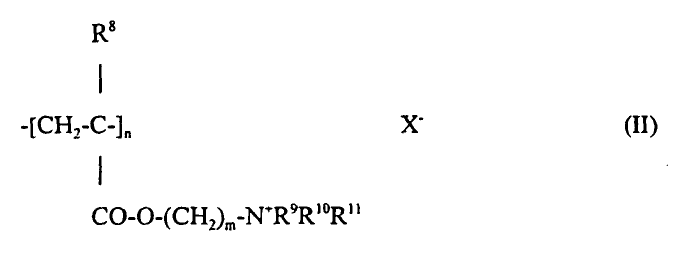 in der R⁸ = -H oder-CH₃ ist, R⁹, R¹⁰ und R¹¹ unabhängig voneinander ausgewählt sind aus C₁₋₄-Alkyl-, -Alkenyl- oder -Hydroxyalkylgruppen, m = 1, 2, 3 oder 4, n eine natürliche Zahl und X- ein physiologisch verträgliches organisches oder anorganisches Anion ist, sowie Copolymere, bestehend im wesentlichen aus den in Formel (II) aufgeführten Monomereinheiten sowie nichtionogenen Monomereinheiten, sind besonders bevorzugte kationische Polymere. Im Rahmen dieser Polymere sind diejenigen erfindungsgemäß bevorzugt, für die mindestens eine der folgenden Bedingungen gilt:
- R⁸ steht für eine Methylgruppe
- R⁹, R¹⁰ und R¹¹ stehen für Methylgruppen
- m hat den Wert 2.

Als physiologisch verträgliches Gegenionen X⁻ kommen beispielsweise Halogenidionen, Sulfationen, Phosphationen, Methosulfationen sowie organische Ionen wie Lactat-, Citrat-, Tartrat- und Acetationen in Betracht. Bevorzugt sind Halogenidionen, insbesondere Chlorid.

Ein besonders geeignetes Homopolymer ist das, gewünschtenfalls vernetzte, Poly(methacryloyloxyethyltrimethylammoniumchlorid) mit der INCI-Bezeichnung Polyquaternium-37. Die Vernetzung kann gewünschtenfalls mit Hilfe mehrfach olefinisch ungesättigter Verbindungen, beispielsweise Divinylbenzol, Tetraallyloxyethan, Methylenbisacrylamid, Diallylether, Polyallylpolyglycerylether, oder Allylethern von Zuckern oder Zuckerderivaten wie Erythritol, Pentaerythritol, Arabitol, Mannitol, Sorbitol, Sucrose oder Glucose erfolgen. Methylenbisacrylamid ist ein bevorzugtes Vernetzungsagens.

Das Homopolymer wird bevorzugt in Form einer nichtwäßrigen Polymerdispersion, die einen Polymeranteil nicht unter 30 Gew.-% aufweisen sollte, eingesetzt. Solche Polymerdispersionen sind unter den Bezeichnungen Salcare^{®} SC 95 (ca. 50 % Polymeranteil, weitere Komponenten: Mineralöl (INCI-Bezeichnung: Mineral Oil) und Tridecyl-polyoxypropylen-polyoxyethylen-ether (INCI-Bezeichnung: PPG-1-Trideceth-6)) und Salcare^{®} SC 96 (ca. 50 % Polymeranteil, weitere Komponenten: Mischung von Diestern des Propylenglykols mit einer Mischung aus Capryl- und Caprinsäure (INCI-Bezeichnung: Propylene Glycol Dicaprylate/Dicaprate) und Tridecyl-polyoxypropylen-polyoxyethylenether (INCI-Bezeichnung: PPG-1-Trideceth-6)) im Handel erhältlich.

Copolymere mit Monomereinheiten gemäß Formel (II) enthalten als nichtionogene Monomereinheiten bevorzugt Acrylamid, Methacrylamid, Acrylsäure-C₁₋₄-alkylester und Methacrylsäure-C₁₋₄-alkylester. Unter diesen nichtionogenen Monomeren ist das Acrylamid besonders bevorzugt. Auch diese Copolymere können, wie im Falle der Homopolymere oben beschrieben, vernetzt sein. Ein erfindungsgemäß bevorzugtes Copolymer ist das vernetzte Acrylamid-Methacryloyloxyethyltrimethylammoniumchlorid-Copolymer. Solche Copolymere, bei denen die Monomere in einem Gewichtsverhältnis von etwa 20:80 vorliegen, sind im Handel als ca. 50 %ige nichtwäßrige Polymerdispersion unter der Bezeichnung Salcare^{®} SC 92 erhältlich.

Weitere bevorzugte kationische Polymere sind beispielsweise
- quaternisierte Cellulose-Derivate, wie sie unter den Bezeichnungen Celquat^{®} und Polymer JR^{®} im Handel erhältlich sind. Die Verbindungen Celquat^{®} H 100, Celquat^{®} L 200 und Polymer JR^{®}400 sind bevorzugte quaternierte Cellulose-Derivate,
- kationische Alkylpolyglycoside gemäß der DE-PS 44 13 686,
- kationisierter Honig, beispielsweise das Handelsprodukt Honeyquat^{®} 50,
- kationische Guar-Derivate, wie insbesondere die unter den Handelsnamen Cosmedia^{®}Guar und Jaguar^{®} vertriebenen Produkte,
- Polysiloxane mit quaternären Gruppen, wie beispielsweise die im Handel erhältlichen Produkte Q2-7224 (Hersteller: Dow Corning; ein stabilisiertes Trimethylsilylamodimethicon), Dow Corning^{®} 929 Emulsion (enthaltend ein hydroxyl-aminomodifiziertes Silicon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Th. Goldschmidt; diquaternäre Polydimethylsiloxane, Quaternium-80),
- polymere Dimethyldiallylammoniumsalze und deren Copolymere mit Estern und Amiden von Acrylsäure und Methacrylsäure. Die unter den Bezeichnungen Merquat^{®}100 (Poly(dimethyldiallylammoniumchlorid)) und Merquat^{®}550 (Dimethyldiallylammoniumchlorid-Acrylamid-Copolymer) im Handel erhältlichen Produkte sind Beispiele für solche kationischen Polymere,
- Copolymere des Vinylpyrrolidons mit quaternierten Derivaten des Dialkylaminoalkylacrylats und -methacrylats, wie beispielsweise mit Diethylsulfat quaternierte Vinylpyrrolidon-Dimethylaminoethylmethacrylat-Copolymere. Solche Verbindungen sind unter den Bezeichnungen Gafquat^{®}734 und Gafquat^{®}755 im Handel erhältlich,
- Vinylpyrrolidon-Vinylimidazoliummethochlorid-Copolymere, wie sie unter den Bezeichnungen Luviquat^{®} FC 370, FC 550, FC 905 und HM 552 angeboten werden.
- quatemierter Polyvinylalkohol,
sowie die unter den Bezeichnungen
- Polyquaternium 2,
- Polyquaternium 17,
- Polyquaternium 18 und
- Polyquaternium 27 bekannten Polymeren mit quartären Stickstoffatomen in der Polymerhauptkette.

Gleichfalls als kationische Polymere eingesetzt werden können die unter den Bezeichnungen Polyquatemium-24 (Handelsprodukt z. B. Quatrisoft^{®} LM 200), bekannten Polymere. Ebenfalls erfindungsgemäß verwendbar sind die Copolymere des Vinylpyrrolidons, wie sie als Handelsprodukte Copolymer 845 (Hersteller: ISP), Gaffix^{®} VC 713 (Hersteller: ISP), Gafquat^{®}ASCP 1011, Gafquat^{®}HS 110, Luviquat^{®}8155 und Luviquat^{®} MS 370 erhältlich sind.

Weitere erfindungsgemäße kationische Polymere (B) sind die sogenannten "temporär kationischen" Polymere. Diese Polymere enthalten üblicherweise eine Aminogruppe, die bei bestimmten pH-Werten als quartäre Ammoniumgruppe und somit kationisch vorliegt. Bevorzugt sind beispielsweise Chitosan und dessen Derivate, wie sie beispielsweise unter den Handelsbezeichnungen Hydagen^{®} CMF, Hydagen^{®} HCMF, Kytamer^{®} PC und Chitolam^{®} NB/101 im Handel frei verfügbar sind.

Erfindungsgemäß bevorzugte kationische Polymere sind kationische Cellulose-Derivate und Chitosan und dessen Derivate, insbesondere die Handelsprodukte Polymer^{®}JR 400, Hydagen^{®} HCMF und Kytamer^{®} PC, kationische Guar-Derivate, kationische Honig-Derivate, insbesondere das Handelsprodukt Honeyquat^{®} 50, kationische Alkylpolyglycodside gemäß der DE-PS 44 13 686 und Polymere vom Typ Polyquaternium-37.

In einer zweiten Ausführungsform werden amphotere Polymere als Bestandteil (B) des Wirkstoffkomplexes eingesetzt. Unter dem Begriff amphotere Polymere werden sowohl solche Polymere, die im Molekül sowohl freie Aminogruppen als auch freie -COOH- oder SO₃H-Gruppen enthalten und zur Ausbildung innerer Salze befähigt sind, als auch zwitterionische Polymere, die im Molekül quartäre Ammoniumgruppen und -COO⁻- oder -SO₃⁻-Gruppen enthalten, und solche Polymere zusammengefaßt, die -COOH- oder SO₃H-Gruppen und quartäre Ammoniumgruppen enthalten.

Ein Beispiel für ein erfindungsgemäß einsetzbares Amphopolymer ist das unter der Bezeichnung Amphomer^{®} erhältliche Acrylharz, das ein Copolymeres aus tert.-Butylaminoethylmethacrylat, N-(1,1,3,3-Tetramethylbutyl)acrylamid sowie zwei oder mehr Monomeren aus der Gruppe Acrylsäure, Methacrylsäure und deren einfachen Estern darstellt.

Weitere erfindungsgemäß einsetzbare amphotere Polymere sind die in der britischen Offenlegungsschrift 2 104 091, der europäischen Offenlegungsschrift 47 714, der europäischen Offenlegungsschrift 217 274, der europäischen Offenlegungsschrift 283 817 und der deutschen Offenlegungsschrift 28 17 369 genannten Verbindungen.

Bevorzugt eingesetzte amphotere Polymere sind solche Polymerisate, die sich im wesentlichen zusammensetzen aus
(a) Monomeren mit quartären Ammoniumgruppen der allgemeinen Formel (III),

   R¹²-CH=CR¹³-CO-Z-(CₙH₂ₙ)-N⁽⁺⁾R¹⁴R¹⁵R¹⁶ A⁽⁻⁾ (III)

   in der R¹² und R¹³ unabhängig voneinander stehen für Wasserstoff oder eine Methylgruppe und R¹⁴, R¹⁵ und R¹⁶ unabhängig voneinander für Alkylgruppen mit 1 bis 4 Kohlenstoff-Atomen. Z eine NH-Gruppe oder ein Sauerstoffatom, n eine ganze Zahl von 2 bis 5 und A⁽⁻⁾ das Anion einer organischen oder anorganischen Säure ist
   und
(b) monomeren Carbonsäuren der allgemeinen Formel (IV),

   R¹⁷-CH=CR¹⁸-COOH (IV)

   in denen R¹⁷ und R¹⁸ unabhängig voneinander Wasserstoff oder Methylgruppen sind.

Diese Verbindungen können sowohl direkt als auch in Salzform, die durch Neutralisation der Polymerisate, beispielsweise mit einem Alkalihydroxid, erhalten wird, erfindungsgemäß eingesetzt werden. Bezüglich der Einzelheiten der Herstellung dieser Polymerisate wird ausdrücklich auf den Inhalt der deutschen Offenlegungsschrift 39 29 973 Bezug genommen. Ganz besonders bevorzugt sind solche Polymerisate, bei denen Monomere des Typs (a) eingesetzt werden, bei denen R¹⁴, R¹⁵ und R¹⁶ Methylgruppen sind, Z eine NH-Gruppe und A⁽⁻⁾ ein Halogenid-, Methoxysulfat- oder Ethoxysulfat-Ion ist; Acrylamidopropyl-trimethyl-ammoruumchlorid ist ein besonders bevorzugtes Monomeres (a). Als Monomeres (b) für die genannten Polymerisate wird bevorzugt Acrylsäure verwendet.

Es ist erfindungsgemäß auch möglich, daß die verwendeten Zubereitungen mehrere, insbesondere zwei verschiedene Polymere (B) enthalten.

Die Polymere (B) sind in den erfindungsgemäß verwendeten Mitteln bevorzugt in Mengen von 0,05 bis 10 Gew.-%, bezogen auf das gesamte Mittel, enthalten. Mengen von 0,1 bis 5, insbesondere von 0,1 bis 3 Gew.-%, sind besonders bevorzugt.

Es wurde weiterhin gefunden, daß die farberhaltende Wirkung des erfindungsgemäßen Wirkstoffkomplexes überraschenderweise noch signifikant gesteigert werden kann, wenn dieser in Kombination mit einem Proteinhydrolysat oder einem Derivat eines Proteinhydrolysats eingesetzt wird.

Proteinhydrolysate sind Produktgemische, die durch sauer, basisch oder enzymatisch katalysierten Abbau von Proteinen (Eiweißen) erhalten werden.

Erfindungsgemäß können Proteinhydrolysate sowohl pflanzlichen als auch tierischen Ursprungs eingesetzt werden.

Tierische Proteinhydrolysate sind beispielsweise Elastin-, Kollagen-, Keratin-, Seiden- und Milcheiweiß-Proteinhydrolysate, die auch in Form von Salzen vorliegen können.

Solche Produkte werden beispielsweise unter den Warenzeichen Dehylan^{®} (Cognis), Promois^{®} (Seiwa Kasei), Collapuron^{®} (Cognis), Nutrilan^{®} (Cognis), Gelita-Sol^{®} (Deutsche Gelatine Fabriken Stoess & Co), Lexein^{®} (Inolex) und Kerasol^{®} (Croda) vertrieben.

Erfindungsgemäß bevorzugt ist die Verwendung von Proteinhydrolysaten pflanzlichen Ursprungs, z. B. Soja-, Mandel-, Erbsen-, Kartoffel- und Weizenproteinhydrolysate. Solche Produkte sind beispielsweise unter den Warenzeichen Gluadin^{®} (Cognis), DiaMin^{®} (Diamalt), Lexein^{®} (Inolex) und Crotein^{®} (Croda) erhältlich.

Wenngleich der Einsatz der Proteinhydrolysate als solche bevorzugt ist, können an deren Stelle gegebenenfalls auch anderweitig erhaltene Aminosäuregemische eingesetzt werden. Ebenfalls möglich ist der Einsatz von Derivaten der Proteinhydrolysate, beispielsweise in Form ihrer Fettsäure-Kondensationsprodukte oder kationisch derivatisiert. Solche Produkte werden beispielsweise unter den Bezeichnungen Lamepon^{®} (Cognis), Lexein^{®} (Inolex), Crolastin^{®} (Croda), Crotein^{®} (Croda), Lamequat^{®} (Cognis) und Croquat^{®} (Croda) vertrieben.

Die Proteinhydrolysate oder deren Derivate sind in den erfindungsgemäß verwendeten Mitteln bevorzugt in Mengen von 0,1 bis 10 Gew.-%, bezogen auf das gesamte Mittel, enthalten. Mengen von 0.1 bis 5 Gew.-% sind besonders bevorzugt.

Ebenfalls als vorteilhaft hat sich die Kombination des farberhaltenden Wirkstoffkomplexes mit kationischen Tensiden erwiesen.

Erfindungsgemäß bevorzugt sind dabei kationische Tenside vom Typ der quartären Ammoniumverbindungen, der Esterquats und der Amidoamine. Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid. Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Solche Produkte werden beispielsweise unter den Warenzeichen Stepantex^{®}, Dehyquart^{®} und Armocare^{®} vertrieben. Die Produkte Armocare^{®} VGH-70, ein N,N-Bis(2-Palmitoyloxyethyl)dimethylammoniumchlorid, sowie Dehyquart^{®} F-75, Dehyquart^{®} C-4046, Dehyquart^{®} L80 und Dehyquart^{®} AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid^{®} S 18 im Handel erhältliche Stearamidopropyl-dimethylamin dar.

Weiterhin vorteilhaft im Sinne der Erfindung sind auch kationisch derivatisierte Proteinhydrolysate, wie sie beispielsweise unter den Warenzeichen Lamequat^{®} und Croquat^{®} im Handel sind.

Die kationischen Tenside oder kationisch derivatisierten Proteinhydrolysate sind in den erfindungsgemäß verwendeten Mitteln bevorzugt in Mengen von 0,05 bis 10 Gew.-%, bezogen auf das gesamte Mittel, enthalten, Mengen von 0,1 bis 5 Gew.-% sind besonders bevorzugt.

Ebenfalls als vorteilhaft hat sich die Kombination des farberhaltenden Wirkstoffkomplexes mit Vitaminen, Provitaminen und Vitaminvorstufen sowie deren Derivaten erwiesen.

Dabei sind erfindungsgemäß solche Vitamine, Pro-Vitamine und Vitaminvorstufen bevorzugt, die üblicherweise den Gruppen A, B, C, E, F und H zugeordnet werden.

Zur Gruppe der als **Vitamin A** bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente kommen erfindungsgemäß beispielsweise Vitamin A-Säure und deren Ester, Vitamin A-Aldehyd und Vitamin A-Alkohol sowie dessen Ester wie das Palmitat und das Acetat in Betracht. Die erfindungsgemäß verwendeten Zubereitungen enthalten die Vitamin A-Komponente bevorzugt in Mengen von 0,05-1 Gew.-%, bezogen auf die gesamte Zubereitung.

Zur **Vitamin B-Gruppe** oder zu dem Vitamin B-Komplex gehören u. a.
- Vitamin B₁ (Thiamin)
- Vitamin B₂ (Riboflavin)
- Vitamin B₃. Unter dieser Bezeichnung werden häufig die Verbindungen Nicotinsäure und Nicotinsäureamid (Niacinamid) geführt. Erfindungsgemäß bevorzugt ist das Nicotinsäureamid, das in den erfindungsgemäß verwendetenen Mitteln bevorzugt in Mengen von 0,05 bis 1 Gew.-%, bezogen auf das gesamte Mittel, enthalten ist.
- Vitamin B₅ (Pantothensäure und Panthenol). Im Rahmen dieser Gruppe wird bevorzugt das Panthenol eingesetzt. Erfindungsgemäß einsetzbare Derivate des Panthenols sind insbesondere die Ester und Ether des Panthenols sowie kationisch derivatisierte Panthenole. Einzelne Vertreter sind beispielsweise das Panthenoltriacetat, der Panthenolmonoethylether und dessen Monoacetat sowie die in der WO 92/13829 offenbarten kationischen Panthenolderivate. Die genannten Verbindungen des Vitamin B₅-Typs sind in den erfindungsgemäß verwendeten Mitteln bevorzugt in Mengen von 0,05 - 10 Gew.-%, bezogen auf das gesamte Mittel, enthalten. Mengen von 0,1 - 5 Gew.-% sind besonders bevorzugt.
- Vitamin B₆ (Pyridoxin sowie Pyridoxamin und Pyridoxal).

**Vitamin C** (Ascorbinsäure). Vitamin C wird in den erfindungsgemäß verwendeten Mitteln bevorzugt in Mengen von 0,1 bis 3 Gew.-%, bezogen auf das gesamte Mittel eingesetzt. Die Verwendung in Form des Palmitinsäureesters, der Glucoside oder der Phosphate kann bevorzugt sein. Die Verwendung in Kombination mit Tocopherolen kann ebenfalls bevorzugt sein.

**Vitamin E** (Tocopherole, insbesondere α-Tocopherol). Tocopherol und seine Derivate, worunter insbesondere die Ester wie das Acetat, das Nicotinat, das Phosphat und das Succinat fallen, sind in den erfindungsgemäß verwendeten Mitteln bevorzugt in Mengen von 0,05-1 Gew.-%, bezogen auf das gesamte Mittel, enthalten.

**Vitamin F.** Unter dem Begriff "Vitamin F" werden üblicherweise essentielle Fettsäuren, insbesondere Linolsäure, Linolensäure und Arachidonsäure, verstanden.

**Vitamin H.** Als Vitamin H wird die Verbindung (3a*S*,4*S*, 6a*R*)-2-Oxohexahydrothienol[3,4-*d*]-imidazol-4-valeriansäure bezeichnet, für die sich aber zwischenzeitlich der Trivialname Biotin durchgesetzt hat. Biotin ist in den erfindungsgemäß verwendeten Mitteln bevorzugt in Mengen von 0,0001 bis 1,0 Gew.-%, insbesondere in Mengen von 0.001 bis 0,01 Gew.-% enthalten.

Bevorzugt enthalten die erfindungsgemäß verwendeten Mittel Vitamine, Provitamine und Vitaminvorstufen aus den Gruppen A, B, E und H.

Panthenol und seine Derivate sowie Nicotinsäureamid und Biotin sind besonders bevorzugt.

Schließlich läßt sich die Wirkung des farberhaltenden Wirkstoffkomplexes auch durch den kombinierten Einsatz mit Pflanzenextrakten steigern.

Üblicherweise werden diese Extrakte durch Extraktion der gesamten Pflanze hergestellt. Es kann aber in einzelnen Fällen auch bevorzugt sein, die Extrakte ausschließlich aus Blüten und/oder Blättern der Pflanze herzustellen.

Hinsichtlich der erfindungsgemäß verwendbaren Pflanzenextrakte wird insbesondere auf die Extrakte hingewiesen, die in der auf Seite 44 der 3. Auflage des Leitfadens zur Inhaltsstoffdeklaration kosmetischer Mittel, herausgegeben vom Industrieverband Körperpflege- und Waschmittel e.V. (IKW), Frankfurt, beginnenden Tabelle aufgeführt sind.

Erfindungsgemäß sind vor allem die Extrakte aus Grünem Tee, Eichenrinde, Brennessel, Hamamelis, Hopfen, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel bevorzugt.

Besonders bevorzugt sind die Extrakte aus Grünem Tee, Eichenrinde, Brennessel, Hamamelis, Hopfen, Kamille, Klettenwurzel, Schachtelhalm, Lindenblüten, Mandel, Aloe Vera, Kokosnuß, Mango, Aprikose, Limone, Weizen, Kiwi, Melone, Orange, Grapefruit, Salbei, Rosmarin, Birke, Wiesenschaumkraut, Quendel, Schafgarbe, Hauhechel, Meristem, Ginseng und Ingwerwurzel.

Ganz besonders für die erfindungsgemäße Verwendung geeignet sind die Extrakte aus Grünem Tee, Mandel, Aloe Vera, Kokosnuß, Mango, Aprikose, Limone, Weizen, Kiwi und Melone.

Als Extraktionsmittel zur Herstellung der genannten Pflanzenextrakte können Wasser. Alkohole sowie deren Mischungen verwendet werden. Unter den Alkoholen sind dabei niedere Alkohole wie Ethanol und Isopropanol, insbesondere aber mehrwertige Alkohole wie Ethylenglykol und Propylenglykol, sowohl als alleiniges Extraktionsmittel als auch in Mischung mit Wasser, bevorzugt. Pflanzenextrakte auf Basis von Wasser/Propylenglykol im Verhältnis 1:10 bis 10:1 haben sich als besonders geeignet erwiesen.

Die Pflanzenextrakte können erfindungsgemäß sowohl in reiner als auch in verdünnter Form eingesetzt werden. Sofern sie in verdünnter Form eingesetzt werden, enthalten sie üblicherweise ca. 2-80 Gew.-% Aktivsubstanz und als Lösungsmittel das bei ihrer Gewinnung eingesetzte Extraktionsmittel oder Extraktionsmittelgemisch.

Weiterhin kann es bevorzugt sein, in den erfindungsgemäßen Mitteln Mischungen aus mehreren, insbesondere aus zwei, verschiedenen Pflanzenextrakten einzusetzen.

Der erfindungsgemäße farberhaltende Wirkstoffkomplex kann prinzipiell dem Färbemittel direkt zugegeben werden. Bevorzugt erfolgt das Aufbringen des farberhaltenden Wirkstoffkomplexes auf die gefärbte keratinische Faser aber in einem getrennten Schritt entweder direkt im Anschluß an den eigentlichen Färbevorgang oder in getrennten Behandlungen, gegebenenfalls auch Tage oder Wochen nach dem Färbevorgang.

Der Begriff Färbevorgang umfaßt dabei alle dem Fachmann bekannten Verfahren, bei denen auf das, gegebenenfalls angefeuchtete, Haar ein Färbemittel aufgebracht wird und dieses entweder für eine Zeit zwischen wenigen Minuten und ca. 45 Minuten auf dem Haar belassen und anschließend mit Wasser oder einem tensidhaltigen Mittel ausgespült wird oder ganz auf dem Haar belassen wird. Es wird in diesem Zusammenhang ausdrücklich auf die bekannten Monographien, z. B. Kh. Schrader, Grundlagen und Rezepturen der Kosmetika, 2. Auflage, Hüthig Buch Verlag, Heidelberg, 1989, verwiesen, die das entsprechende Wissen des Fachmannes wiedergeben.

Hinsichtlich der Art, gemäß der der erfindungsgemäße farberhaltende Wirkstoffkomplex auf die keratinische Faser, insbesondere das menschliche Haar, aufgebracht wird, bestehen keine prinzipiellen Einschränkungen. Als Konfektionierung dieser Zubereitungen sind beispielsweise Cremes. Lotionen, Lösungen, Wässer, Emulsionen wie W/O-, O/W-, PIT (Emulsionen nach der Lehre der Phaseninversion, PIT genannt), Mikroemulsionen und multiple Emulsionen, Gele, Sprays, Aerosole und Schaumaerosole geeignet. Diese werden in der Regel auf wäßriger oder wäßrig-alkoholischer Basis formuliert. Als alkoholische Komponente kommen dabei niedere Alkanole sowie Polyole wie Propylenglykol und Glycerin zum Einsatz. Ethanol und Isopropanol sind bevorzugte Alkohole. Wasser und Alkohol können in der wäßrig alkoholischen Basis in einem Gewichtsverhältnis von 1 : 10 bis 10 : 1 vorliegen. Wasser sowie wäßrig-alkoholische Mischungen, die bis zu 50 Gew.%, insbesondere bis zu 25 Gew.%, Alkohol, bezogen auf das Gemisch Alkohol/Wasser, enthalten, können erfindungsgemäß bevorzugte Grundlagen sein. Der pH-Wert dieser Zubereitungen kann prinzipiell bei Werten von 2 - 11 liegen. Er liegt bevorzugt zwischen 2 und 7, wobei Werte von 3 bis 5 besonders bevorzugt sind. Zur Einstellung dieses pH-Wertes kann praktisch jede für kosmetische Zwecke verwendbare Säure oder Base verwendet werden. Üblicherweise werden als Säuren Genußsäuren verwendet. Unter Genußsäuren werden solche Säuren verstanden, die im Rahmen der üblichen Nahrungsaufnahme aufgenommen werden und positive Auswirkungen auf den menschlichen Organismus haben. Genußsäuren sind beispielsweise Essigsäure, Milchsäure, Weinsäure, Zitronensäure, Äpfelsäure, Ascorbinsäure und Gluconsäure. Im Rahmen der Erfindung ist die Verwendung von Zitronensäure und Milchsäure besonders bevorzugt. Bevorzugte Basen sind Ammoniak, Alkalihydroxide, Triethanolamin sowie N,N,N',N'-Tetrakis-(2-hydroxypropyl)-ethylendiamin.

Auf dem Haar verbleibende Zubereitungen haben sich als besonders wirksam erwiesen und können daher bevorzugte Ausführungsformen der erfindungsgemäßen Lehre darstellen. Unter auf dem Haar verbleibend werden erfindungsgemäß solche Zubereitungen verstanden, die nicht im Rahmen der Behandlung nach einem Zeitraum von wenigen Sekunden bis zu einer Stunde mit Hilfe von Wasser oder einer wäßrigen Lösung wieder aus dem Haar ausgespült werden. Vielmehr verbleiben die Zubereitungen bis zur nächsten Haarwäsche, d. h. in der Regel mehr als 12 Stunden, auf dem Haar.

Gemäß einer bevorzugten Ausführungsform werden diese Zubereitungen als Haarkur oder Haar-Conditioner formuliert. Die erfindungsgemäßen Zubereitungen gemäß dieser Ausführungsform können nach Ablauf dieser Einwirkzeit mit Wasser oder einem zumindest überwiegend wasserhaltigen Mittel ausgespült werden; bevorzugt werden sie jedoch, wie oben ausgerührt, auf dem Haar belassen. Dabei kann es bevorzugt sein, die erfindungsgemäße Zubereitung vor der Anwendung eines reinigenden Mittels, eines Wellmittels oder anderen Haarbehandlungsmitteln auf das Haar aufzubringen. In diesem Falle dient die erfindungsgemäße Zubereitung als Farbschutz für die nachfolgenden Anwendungen.

Gemäß weiteren Ausführungsformen kann es sich bei den erfindungsgemäßen Mitteln aber beispielsweise auch um reinigende Mittel wie Shampoos, pflegende Mittel wie Spülungen, festigende Mittel wie Haarfestiger, Schaumfestiger, Styling Gels und Fönfestiger, dauerhafte Verformungsmittel wie Dauerwell- und Fixiermittel sowie insbesondere im Rahmen eines Dauerwellverfahrens oder Färbeverfahrens eingesetzte Vorbehandlungsmittel oder Nachspülungen handeln.

Neben dem erfindungsgemäß zwingend erforderlichen farberhaltenden Wirkstoffkomplex und den weiteren, oben genannten bevorzugten Komponenten können diese Zubereitungen prinzipiell alle weiteren, dem Fachmann für solche kosmetischen Mittel bekannten Komponenten enthalten.

Weitere Wirk-, Hilfs- und Zusatzstoffe sind beispielsweise
- nichtionogene Tenside wie beispielsweise Alkylphenolpolyglycolether, Fettsäurepolyglycolester, Fettsäureamidpolyglycolether, Fettaminpolyglycolether, alkoxylierte Triglyceride, wie insbesondere ethoxyliertes Rizinusöl, Alk(en)yloligoglucoside, Fettsäure-N-alkylglucamide, Polyolfettsäureester, Zuckerester, Sorbitanester und Polysorbate. Sofern die nichtionischen Tenside Polyglycoletherketten enthalten, können sie eine konventionelle oder eingeengte Homologenverteilüng aufweisen.
- anionische Tenside, insbesondere Alkylsulfate, Alkylpolyglykolethersulfate und Ethercarbonsäuren mit 10 bis 18 C-Atomen in der Alkylgruppe und bis zu 12 Glykolethergruppen im Molekül. Seifen sowie Sulfobernsteinsäuremono- und -dialkylester mit 8 bis 18 C-Atomen in der Alkylgruppe und Sulfobernsteinsäuremono-alkylpolyoxyethyl-ester mit 8 bis 18 C-Atomen in der Alkylgruppe und 1 bis 6 Oxyethylgruppen.
- zwitterionische Tenside, insbesondere die sogenannten Betaine wie die N-Alkyl-N,N-dimethylammonium-glycinate, beispielsweise das Kokosalkyl-dimethylammonium-glycinat, N-Acyl-aminopropyl-N,N-dimethylammoniumglycinate, beispielsweise das Kokosacylaminopropyl-dimethylammoniumglycinat, und 2-Alkyl-3-carboxylmethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat,
- ampholytische Tenside wie beispielsweise N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkyl-aminoessigsäuren mit jeweils etwa 8 bis 18 C-Atomen in der Alkylgruppe,
- nichtionische Polymere wie beispielsweise Vinylpyrrolidon/Vinylacrylat-Copoly mere, Polyvinylpyrrolidon und Vinylpyrrolidon/Vinylacetat-Copolymere und Polysiloxane,
- anionische Polymere wie beispielsweise Acrylsäure - Acrylamid Copolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z. B. Polyvinylalkohol,
- Strukturanten wie Maleinsäure und Milchsäure,
- haarkonditionierende Verbindungen wie Phospholipide, beispielsweise Sojalecithin, Ei-Lecitin und Kephaline, sowie Silikonöle,
- Parfümöle, Dimethylisosorbid und Cyclodextrine,
- Lösungsmittel und -vermittler wie Ethanol. Isopropanol, Ethylenglykol, Propylenglykol. Glycerin und Diethylenglykol,
- symmetrische und unsymmetrische, lineare und verzweigte Dialkylether mit insgesamt zwischen 12 bis 36 C-Atomen, insbesondere 12 bis 24 C-Atomen, wie beispielsweise Di-n-octylether, Di-n-decylether. Di-n-nonylether, Di-n-undecylether und Di-n-dodecylether, n-Hexyl-n-octylether, n-Octyl-n-decylether, n-Decyl-n-undecylether, n-Undecyl-n-dodecylether und n-Hexyl-n-Undecylether sowie Di-tert-butylether, Di-iso-pentylether, Di-3-ethyldecylether, tert.-Butyl-n-octylether, isoPentyl-n-octylether und 2-Methyl-pentyl-n-octylether,
- Fettalkohole, insbesondere lineare und/oder gesättigte Fettalkohole mit 8 bis 30 C-Atomen, und Monoester der Fettsäuren mit Alkoholen mit 6 bis 24 C-Atomen,
- faserstrukturverbessernde Wirkstoffe, insbesondere Mono-, Di- und Oligosaccharide wie beispielsweise Glucose, Galactose, Fructose, Fruchtzucker und Lactose,
- konditionierende Wirkstoffe wie Paraffinöle, pflanzliche Öle, z. B. Sonnenblumenöl, Orangenöl, Mandelöl, Weizenkeimöl und Pfirsichkernöl sowie Phospholipide, beispielsweise Sojalecithin, Ei-Lecithin und Kephaline,
- quaternierte Amine wie Methyl-1-alkylamidoethyl-2-alkylimidazolinium-methosulfat
- Entschäumer wie Silikone,
- Farbstoffe zum Anfärben des Mittels,
- Antischuppenwirkstoffe wie Piroctone Olamine, Zink Omadine und Climbazol,
- Lichtschutzmittel, insbesondere derivatisierte Benzophenone, Zimtsäure-Derivate und Triazine,
- weitere Substanzen zur Einstellung des pH-Wertes, wie beispielsweise α- und β-Hydroxycarbonsäuren
- Wirkstoffe wie Allantoin und Bisabolol,
- Cholesterin,
- Konsistenzgeber wie Zuckerester, Polyolester oder Polyolalkylether,
- Fette und Wachse wie Walrat, Bienenwachs, Montanwachs und Paraffine,
- Fettsäurealkanolamide,
- Komplexbildner wie EDTA, NTA, β-Alanindiessigsäure und Phosphonsäuren,
- Quell- und Penetrationsstoffe wie Glycerin, Propylenglykolmonoethylether, Carbonate. Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Pigmente,
- Reduktionsmittel wie z. B. Thioglykolsäure und deren Derivate, Thiomilchsäure, Cysteamin, Thioäpfelsäure und α-Mercaptoethansulfonsäure,
- Treibmittel wie Propan-Butan-Gemische, N₂O, Dimethylether, CO₂ und Luft,
- Antioxidantien.

Bezüglich weiterer fakultativer Komponenten sowie die eingesetzten Mengen dieser Komponenten wird ausdrücklich auf die dem Fachmann bekannten einschlägigen Handbücher, z. B. die oben genannte Monographie von Kh. Schrader verwiesen.

Wie bereits oben erwähnt ist es im Rahmen der erfindungsgemäßen Lehre auch möglich, wenngleich weniger bevorzugt, den farberhaltenden Wirkstoffkomplex direkt in die Färbe- oder Tönungsmittel einzuarbeiten.

Die Zusammensetzung des Färbe- oder Tönungsmittels unterliegt keinen prinzipiellen Einschränkungen.
Als Farbstoff(vorprodukt)e können
- Oxidationsfarbstoffvorprodukte vom Entwickler- und Kuppler-Typ,
- natürliche und synthetische direktziehende Farbstoffe und
- Vorstufen naturanaloger Farbstoffe, wie Indol- und Indolin-Derivate,
sowie Mischungen von Vertretern einer oder mehrerer dieser Gruppen eingesetzt werden.

Als Oxidationsfarbstoffvorprodukte vom Entwickler-Typ werden üblicherweise primäre aromatische Amine mit einer weiteren, in para- oder ortho-Position befindlichen, freien oder substituierten Hydroxy- oder Aminogruppe, Diaminopyridinderivate, heterocyclische Hydrazone, 4-Aminopyrazolderivate sowie 2,4,5,6-Tetraaminopyrimidin und dessen Derivate eingesetzt. Geeignete Entwicklerkomponenten sind beispielsweise p-Phenylendiamin, p-Toluylendiamin, p-Aminophenol, o-Aminophenol, 1-(2'-Hydroxyethyl)-2.5-diaminobenzol, N,N-Bis-(2-hydroxy-ethyl)-p-phenylendiamin, 2-(2,5-Diaminophenoxy)-ethanol, 4-Amino-3-methylphenol, 2,4,5,6-Tetraaminopyrimidin, 2-Hydroxy-4,5,6-triaminopyrimidin, 4-Hydroxy-2,5,6-triaminopyrimidin, 2,4-Dihydroxy-5,6-diaminopyrimidin, 2-Dimethylamino-4,5,6-triaminopyrimidin, 2-Hydroxymethylamino-4-amino-phenol, Bis-(4-aminophenyl)amin, 4-Amino-3-fluorphenol, 2-Aminomethyl-4-aminophenol, 2-Hydroxymethyl-4-aminophenol, 4-Amino-2-((diethylamino)-methyl)-phenol, Bis-(2-hydroxy-5-aminophenyl)-methan, 1,4-Bis-(4-aminophenyl)-diazacycloheptan, 1,3-Bis(N(2-hydroxyethyl)-N(4-aminophenylamino))-2-propanol, 4-Amino-2-(2-hydroxyethoxy)-phenol, 1,10-Bis-(2,5-diaminophenyl)-1,4,7,10-tetraoxadecan sowie 4,5-Diaminopyrazol-Derivate nach EP 0 740 741 bzw. WO 94/08970 wie z. B. 4,5-Diamino-1-(2'-hydroxyethyl)-pyrazol. Besonders vorteilhafte Entwicklerkomponenten sind p-Phenylendiamin, p-Toluylendiamin, p-Aminophenol, 1-(2'-Hydroxyethyl)-2,5-diaminobenzol, 4-Amino-3-methylphenol, 2-Aminomethyl-4-aminophenol, 2,4,5,6-Tetraaminopyrimidin, 2-Hydroxy-4,5,6-triaminopyrimidin, 4-Hydroxy-2,5,6-triaminopyrimidin.

Als Oxidationsfarbstoffvorprodukte vom Kuppler-Typ werden in der Regel m-Phenylendiaminderivate, Naphthole, Resorcin und Resorcinderivate, Pyrazolone und m-Aminophenolderivate verwendet. Beispiele für solche Kupplerkomponenten sind
- m-Aminophenol und dessen Derivate wie beispielsweise 5-Amino-2-methylphenol, 5-(3-Hydroxypropylamino)-2-methylphenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)-amino-2-methylphenol, 3-(Diethylamino)-phenol, N-Cyclopentyl-3-aminophenol, 1,3-Dihydroxy-5-(methylamino)-benzol, 3-(Ethylamino)-4-methylphenol und 2,4-Dichlor-3-aminophenol,
- o-Aminophenol und dessen Derivate,
- m-Diaminobenzol und dessen Derivate wie beispielsweise 2,4-Diaminophenoxyethanol, 1,3-Bis-(2,4-diaminophenoxy)-propan, 1-Methoxy-2-amino-4-(2'-hydroxyethylamino)benzol, 1,3-Bis-(2,4-diaminophenyl)-propan, 2,6-Bis-(2-hydroxyethylamino)-1-methylbenzol und 1-Amino-3-bis-(2'-hydroxyethyl)-aminobenzol,
- o-Diaminobenzol und dessen Derivate wie beispielsweise 3,4-Diaminobenzoesäure und 2.3-Diamino-1-methylbenzol.
- Di- beziehungsweise Trihydroxybenzolderivate wie beispielsweise Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol,
- Pyridinderivate wie beispielsweise 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin und 3,5-Diamino-2,6-dimethoxypyridin,
- Naphthalinderivate wie beispielsweise 1-Naphthol, 2-Methyl-1-naphthol, 2-Hydroxymethyl-1-naphthol, 2-Hydroxyethyl-1-naphthol, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin,
- Morpholinderivate wie beispielsweise 6-Hydroxybenzomorpholin und 6-Amino-benzomorpholin,
- Chinoxalinderivate wie beispielsweise 6-Methyl-1,2,3,4-tetrahydrochinoxalin,
- Pyrazolderivate wie beispielsweise 1-Phenyl-3-methylpyrazol-5-on,
- Indolderivate wie beispielsweise 4-Hydroxyindol, 6-Hydroxyindol und 7-Hydroxyindol,
- Methylendioxybenzolderivate wie beispielsweise 1-Hydroxy-3,4-methylendioxybenzol, 1-Amino-3,4-methylendioxybenzol und 1-(2'-Hydroxyethyl)-amino-3,4-methylendioxybenzol,

Besonders geeignete Kupplerkomponenten sind 1-Naphthol, 1,5-, 2,7- und 1,7-Dihydroxynaphthalin, 3-Aminophenol, 5-Amino-2-methylphenol, 2-Amino-3-hydroxypyridin, Resorcin, 4-Chlorresorcin, 2-Chlor-6-methyl-3-aminophenol, 2-Methylresorcin, 5-Methylresorcin, 2,5-Dimethylresorcin und 2,6-Dihydroxy-3,4-dimethylpyridin.

Direktziehende Farbstoffe sind üblicherweise Nitrophenylendiamine, Nitroaminophenole, Azofarbstoffe. Anthrachinone oder Indophenole. Besonders geeignete direktziehende Farbstoffe sind die unter den internationalen Bezeichnungen bzw. Handelsnamen HC Yellow 2, HC Yellow 4, HC Yellow 5, HC Yellow 6, Basic Yellow 57, Disperse Orange 3, HC Red 3, HC Red BN, Basic Red 76, HC Blue 2, HC Blue 12, Disperse Blue 3, Basic Blue 99, HC Violet 1, Disperse Violet 1, Disperse Violet 4, Disperse Black 9, Basic Brown 16 und Basic Brown 17 bekannten Verbindungen sowie 1,4-Bis-(β-hydroxyethyl)-amino-2-nitrobenzol, 4-Amino-2-nitrodiphenylamin-2'-carbonsäure, 6-Nitro-1,2,3,4-tetrahydrochinoxalin, Hydroxyethyl-2-nitro-toluidin, Pikraminsäure, 2-Amino-6-chloro-4-nitrophenol, 4-Ethylamino-3-nitrobenzoesäure und 2-Chloro-6-ethylamino-1-hydroxy-4-nitrobenzol.

In der Natur vorkommende direktziehende Farbstoffe sind beispielsweise Henna rot, Henna neutral, Henna schwarz, Kamillenblüte, Sandelholz, schwarzen Tee, Faulbaumrinde, Salbei, Blauholz, Krappwurzel, Catechu, Sedre und Alkannawurzel enthalten.

Es ist nicht erforderlich, daß die Oxidationsfarbstoffvorprodukte oder die direktziehenden Farbstoffe jeweils einheitliche Verbindungen darstellen. Vielmehr können in den erfindungsgemäßen Haarfärbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z. B. toxikologischen, ausgeschlossen werden müssen.

Bezüglich der in den erfindungsgemäßen Haarfärbe- und -tönungsmitteln einsetzbaren Farbstoffe wird weiterhin ausdrücklich auf die Monographie Ch. Zviak, The Science of Hair Care, Kapitel 7 (Seiten 248-250; direktziehende Farbstoffe) sowie Kapitel 8, Seiten 264-267; Oxidationsfarbstoffvorprodukte), erschienen als Band 7 der Reihe "Dermatology" (Hrg.: Ch.. Culnan und H. Maibach), Verlag Marcel Dekker Inc.. New York, Basel. 1986. sowie das "Europäische Inventar der Kosmetik-Rohstoffe", herausgegeben von der Europäischen Gemeinschaft, erhältlich in Diskettenform vom Bundesverband Deutscher Industrie- und Handelsunternehmen für Arzneimittel, Reformwaren und Körperpflegemittel e.V., Mannheim. Bezug genommen.

Als Vorstufen naturanaloger Farbstoffe werden beispielsweise Indole und Indoline sowie deren physiologisch verträgliche Salze verwendet. Bevorzugt werden solche Indole und Indoline eingesetzt, die mindestens eine Hydroxy- oder Aminogruppe, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe. Besonders vorteilhafte Eigenschaften haben 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbonsäure, 6-Hydroxyindolin, 6-Aminoindolin und 4-Aminoindolin sowie 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, 6-Hydroxyindol, 6-Aminoindol und 4-Aminoindol.

Besonders hervorzuheben sind innerhalb dieser Gruppe N-Methyl-5,6-dihydroxyindolin, N-Ethyl-5,6-dihydroxyindolin, N-Propyl-5,6-dihydroxyindolin, N-Butyl-5,6-dihydroxyindolin und insbesondere das 5,6-Dihydroxyindolin sowie N-Methyl-5,6-dihydroxyindol, N-Ethyl-5,6-dihydroxyindol, N-Propyl-5,6-dihydroxyindol, N-Butyl-5,6-dihydroxyindol sowie insbesondere das 5,6-Dihydroxyindol.

Die Indolin- und Indol-Derivate in den im Rahmen des erfindungsgemäßen Verfahrens eingesetzten Färbemitteln sowohl als freie Basen als auch in Form ihrer physiologisch verträglichen Salze mit anorganischen oder organischen Säuren, z. B. der Hydrochloride, der Sulfate und Hydrobromide, eingesetzt werden.

Bei der Verwendung von Farbstoff-Vorstufen vom Indolin- oder Indol-Typ kann es bevorzugt sein, diese zusammen mit mindestens einer Aminosäure und/oder mindestens einem Oligopeptid einzusetzen. Bevorzugte Aminosäuren sind Aminocarbonsäuren, insbesondere α-Aminocarbonsäuren und ω-Aminocarbonsäuren. Unter den α-Aminocarbonsäuren sind wiederum Arginin, Lysin, Omithin und Histidin besonders bevorzugt. Eine ganz besonders bevorzugte Aminosäure ist Arginin, insbesondere in freier Form, aber auch als Hydrochlorid eingesetzt.

Haarfärbemittel, insbesondere wenn die Ausfärbung oxidativ, sei es mit Luftsauerstoff oder anderen Oxidationsmitteln wie Wasserstoffperoxid, erfolgt, werden üblicherweise schwach sauer bis alkalisch, d. h. auf pH-Werte im Bereich von etwa 5 bis 11, eingestellt. Zu diesem Zweck enthalten die Färbemittel Alkalisierungsmittel, üblicherweise Alkali- oder Erdalkalihydroxide, Ammoniak oder organische Amine. Bevorzugte Alkalisierungsmittel sind Monoethanolamin, Monoisopropanolamin, 2-Amino-2-methyl-propanol, 2-Amino-2-methyl-1,3-propandiol, 2-Amino-2-ethyl-1,3-propandiol, 2-Amino-2-methylbutanol und Triethanolamin sowie Alkali- und Erdalkalimetallhydroxide. Insbesondere Monoethanolamin, Triethanolamin sowie 2-Amino-2-methyl-propanol und 2-Amino-2-methyl-1,3-propandiol sind im Rahmen dieser Gruppe bevorzugt. Auch die Verwendung von ω-Aminosäuren wie ω-Aminocapronsäure als Alkalisierungsmittel ist möglich.

Erfolgt die Ausbildung der eigentlichen Haarfarben im Rahmen eines oxidativen Prozesses, so können übliche Oxidationsmittel, wie insbesondere Wasserstoffperoxid oder dessen Anlagerungsprodukte an Harnstoff, Melamin oder Natriumborat verwendet werden. Die Oxidation mit Luftsauerstoff als einzigem Oxidationsmittel kann allerdings bevorzugt sein. Weiterhin ist es möglich, die Oxidation mit Hilfe von Enzymen durchzuführen, wobei die Enzyme sowohl zur Erzeugung von oxidierenden Per-Verbindungen eingesetzt werden als auch zur Verstärkung der Wirkung einer geringen Menge vorhandener Oxidationsmittel. So können die Enzyme (Enzymklasse 1: Oxidoreduktasen) Elektronen aus geeigneten Entwicklerkomponenten (Reduktionsmittel) auf Luftsauerstoff übertragen. Bevorzugt sind dabei Oxidasen wie Tyrosinase und Laccase aber auch Glucoseoxidase, Uricase oder Pyruvatoxidase. Weiterhin sei das Vorgehen genannt, die Wirkung geringer Mengen (z. B. 1% und weniger, bezogen auf das gesamte Mittel) Wasserstoffperoxid durch Peroxidasen zu verstärken.

Zweckmäßigerweise wird die Zubereitung des Oxidationsmittels dann unmittelbar vor dem Färben der Haare mit der Zubereitung mit den Farbstoffvorprodukten vermischt. Das dabei entstehende gebrauchsfertige Haarfärbepräparat sollte bevorzugt einen pH-Wert im Bereich von 6 bis 10 aufweisen. Besonders bevorzugt ist die Anwendung der Haarfärbemittel in einem schwach alkalischen Milieu. Die Anwendungstemperaturen können in einem Bereich zwischen 15 und 40 °C, bevorzugt bei der Temperatur der Kopfhaut, liegen. Nach einer Einwirkungszeit von ca. 5 bis 45, insbesondere 15 bis 30, Minuten wird das Haarfärbemittel durch Ausspülen von dem zu färbenden Haar entfernt. Das Nachwaschen mit einem Shampoo entfällt, wenn ein stark tensidhaltiger Träger, z. B. ein Färbeshampoo, verwendet wurde.

Insbesondere bei schwer färbbarem Haar kann die Zubereitung mit den Farbstoffvorprodukten ohne vorherige Vermischung mit der Oxidationskomponente auf das Haar aufgebracht werden. Nach einer Einwirkdauer von 20 bis 30 Minuten wird dann - gegebenenfalls nach einer Zwischenspülung - die Oxidationskomponente aufgebracht. Nach einer weiteren Einwirkdauer von 10 bis 20 Minuten wird dann gespült und gewünschtenfalls nachshampooniert. Bei dieser Ausführungsform wird gemäß einer ersten Variante, bei der das vorherige Aufbringen der Farbstoffvorprodukte eine bessere Penetration in das Haar bewirken soll, das entsprechende Mittel auf einen pH-Wert von etwa 4 bis 7 eingestellt. Gemäß einer zweiten Variante wird zunächst eine Luftoxidation angestrebt, wobei das aufgebrachte Mittel bevorzugt einen pH-Wert von 7 bis 10 aufweist. Bei der anschließenden beschleunigten Nachoxidation kann die Verwendung von sauer eingestellten Peroxidisulfat-Lösungen als Oxidationsmittel bevorzugt sein.

Weiterhin kann die Ausbildung der Färbung dadurch unterstützt und gesteigert werden, daß dem Mittel bestimmte Metallionen zugesetzt werden. Solche Metallionen sind beispielsweise Zn²⁺, Cu²⁺, Fe²⁺, Fe³⁺, Mn²⁺, Mn⁴⁺, Li⁺, Mg²⁺, Ca²⁺ und Al³⁺. Besonders geeignet sind dabei Zn²⁺, Cu²⁺ und Mn²⁺. Die Metallionen können prinzipiell in der Form eines beliebigen, physiologisch verträglichen Salzes eingesetzt werden. Bevorzugte Salze sind die Acetate, Sulfate. Halogenide, Lactate und Tartrate. Durch Verwendung dieser Metallsalze kann sowohl die Ausbildung der Färbung beschleunigt als auch die Farbnuance gezielt beeinflußt werden.

Ein zweiter Gegenstand der Erfindung sind Mittel zur Verbesserung der Waschechtheit gefärbter Fasern, insbesondere keratinischer Fasern, die eine Kombination aus
- einer Pyrolidinoncarbonsäure gemäß Formel (I),
- einem Polymer und
- einem Proteinhydrolysat und/oder Derivat eines Proteinhydrolysates enthält.

Dabei sind solche Mittel bevorzugt, die pflanzliche Proteinhydrolysate enthalten.

Ein dritter Gegenstand der Erfindung ist ein Verfahren zur Färbung von Fasern, insbesondere keratinischer, Fasern, bei dem in einem ersten Schritt die Fasern in üblicher Weise gefärbt werden und in einem zweiten Schritt ein Mittel auf die Fasern aufgetragen wird, das den Wirkstoffkomplex gemäß einem der Ansprüche 1 bis 13 zu Erhöhung der Waschechtheit der Färbung enthält, wobei das Mittel gewünschtenfalls nach einer Einwirkzeit von 1 bis 5 Minuten wieder ausgespült wird.

Ein vierter Gegenstand der Erfindung schließlich ist ein Verfahren zur Färbung von Fasern, insbesondere keratinischer Fasern, bei dem in einem ersten Schritt ein Mittel auf die Fasern aufgetragen wird, das den Wirkstoffkomplex gemäß einem der Ansprüche 1 bis 13 zur Erhöhung der Waschechtheit der Färbung enthält, und anschließend in einem zweiten Schritt die Fasern in üblicher Weise gefärbt werden. Im Rahmen dieses Verfahrens kann es bevorzugt sein, das erste Mittel in Form eines Sprays zu applizieren.

### Beispiele

Alle Mengenangaben sind, soweit nicht anders vermerkt, Gewichtsteile.

### 1. Haarspülung

| | |
|---|---|
| Eumulgin^{®} B2¹ | 0,3 |
| Cetyl/Stearylalkohol | 3,3 |
| Isopropylmyristat | 0,5 |
| Paraffinöl perliquidum 15 cSt. DAB 9 | 0,3 |
| Dehyquart^{®}A-CA² | 2,0 |
| Salcare^{®}SC 96³ | 1,0 |
| Citronensäure | 0,4 |
| Ajidew^{®} NL 50⁴ | 2,0 |
| Phenonip^{®5} | 0,8 |
| Wasser | ad 100 |

| | |
|---|---|
| ¹ Cetylstearylalkohol + 20 EO (INCI-Bezeichnung: Ceteareth-20) (COGNIS) ² Trimethylhexadecylammoniumchlorid ca. 25% Aktivsubstanz (INCI-Bezeichnung: Cetrimonium Chloride) (COGNIS) ³ N,N,N-Trimethyl-2[(methyl-1-oxo-2-propenyl)oxy]-Ethanaminiumchlorid-Homopolymer (50 % Aktivsubstanz; INCI-Bezeichnung: Polyquaternium-37 (and) Propylenglycol Dicaprilate Dicaprate (and) PPG-1 Trideceth-6) (ALLIED COLLOIDS) ⁴ Natriumsalz der 2-Pyrrolidinon-5-carbonsäure ⁵ Hydroxybenzoesäuremethylester-Hydroxybenzoesäureethylester-Hydroxybenzoesäurepropylester-Hydroxybenzoesäurebutylester-Phenoxyethanol-Gemisch (ca. 28 % Aktivsubstanz; INCI-Bezeichnung: Phenoxyethanol, Methylparaben, Ethylparaben, Propylparaben. Butylparaben) (NIPA) | |

### 2. Haarspülung

| | |
|---|---|
| Eumulgin^{®} B2 | 0,3 |
| Cetyl/Stearylalkohol | 3,3 |
| Isopropylmyristat | 0,5 |
| Paraffinöl perliquidum 15 cSt. DAB 9 | 0,3 |
| Dehyquart^{®}L 80⁶ | 0,4 |
| Cosmedia Guar^{®} C 261⁷ | 1,5 |
| Ajidew^{®} NL 50 | 3,0 |
| Citronensäure | 0,4 |
| Phenonip^{®} | 0,8 |
| Wasser | ad 100 |

| | |
|---|---|
| ⁶ Bis(cocoylethyl)-hydroxyethyl-methyl-ammonium-methosulfat (ca. 76 % Aktivsubstanz in Propylenglykol; INCI-Bezeichnung: Dicocoylethyl Hydroxyethylmonium Methosulfat, Propylene Glycol) (COGNIS) ⁷ Guarhydroxypropyltrimethylammonium Chlorid; INCI-Bezeichnung: Guar Hydroxypropyl Trimonium Chloride (COGNIS) | |

### 3. Haarkur

| | |
|---|---|
| Dehyquart^{®} F75⁸ | 4,0 |
| Cetyl/Stearylalkohol | 4,0 |
| Paraffinöl perliquidum 15 cSt DAB 9 | 1,5 |
| Dehyquart^{®}A-CA | 4,0 |
| Salcare^{®}SC 96 | 1,5 |
| (R)-(+)-2-Pyrrolidinon-5-carbonsäure (Fa. Merck), neutralisiert mit NaOH (50% wäßrige Lösung) | 1,0 |
| Gluadin^{®}W 20⁹ | 3,0 |
| Citronensäure | 0,15 |
| Phenonip^{®} | 0,8 |
| Wasser | ad 100 |

| | |
|---|---|
| ⁸ Fettalkohole-Methyltriethanolammoniummethylsulfatdialkylester-Gemisch (INCI-Bezeichnung: Distearoylethyl Hydroxyethylmonium Methosulfate, Cetearyl Alcohol) (COGNIS) ⁹ Weizenproteinhydrolysat (20 % Aktivsubstanz in Wasser; INCI-Bezeichnung: Aqua (and) Hydrolized Wheat Protein (and) Sodium Benzoate (and) Phenoxyethanol (and) Methylparaben (and) Propylparaben) (COGNIS) | |

### 4. Haarkur

| | |
|---|---|
| Dehyquart^{®} L80 | 2,0 |
| Cetyl/Stearylalkohol | 6,0 |
| Paraffinöl perliquidum 15 cSt DAB 9 | 2,0 |
| Rewoquat^{®}W 75 PG¹⁰ | 2,0 |
| Cosmedia Guar^{®} C261 | 0,5 |
| Sepigel^{®}305¹¹ | 3,5 |
| Honeyquat^{®} 50¹² | 1,0 |
| Ajidew^{®} NL 50 | 2,5 |
| Gluadin^{®}W 20 | 3,0 |
| Citronensäure | 0,15 |
| Phenonip^{®} | 0,8 |
| Wasser | ad 100 |

| | |
|---|---|
| ¹⁰ 1-Methyl-2-nortalgalkyl-3-talgfettsäureamidoethylimidazolinium-methosulfat (ca. 75 % Aktivsubstanz in Propylenglykol; INCI-Bezeichnung: Quaternium-27, Propylene Glycol) (WITCO) ¹¹ Copolymer aus Acrylamid und 2-Acrylamido-2-methylpropansulfonsäure (INCI-Bezeichnung: Polyacrylamide (and) C₁₃-C₁₄ Isoparaffin (and) Laureth-7) (SEPPIC) ¹² INCI - Bezeichnung: Hydroxypropyltrimonium Honey (BROOKS) | |

### 5. Haarkur

| | |
|---|---|
| Dehyquart^{®} F75 | 0,3 |
| Salcare^{®}SC 96 | 5,0 |
| (S)-(-)-2-Pyrrolidinon-5-carbonsäure (Fa. Merck), neutralisiert mit KOH (50% wäßrige Lösung) | 1,5 |
| Dow Corning^{®}200 Fluid, 5 cSt.¹³ | 1,5 |
| Gafquat^{®}755N¹⁴ | 1,5 |
| Biodocarb^{® 15} | 0,02 |
| Parfümöl | 0,25 |
| Wasser | ad 100 |

| | |
|---|---|
| ¹³ Polydimethylsiloxan (INCI-Bezeichnung: Dimethicone) (DOW CORNING) ¹⁴ Dimethylaminoethylmethacrylat-Vinylpyrrolidon-Copolymer, mit Diethylsulfat quaterniert (19 % Aktivsubstanz in Wasser; INCI-Bezeichnung: Polyquaternium-11) (GAF) ¹⁵ 3-Iod-2-propinyl-n-butylcarbamat (INCI-Bezeichnung: Iodopropynyl Butylcarbamate) (MILKER & GRÜNING) | |

### 6. Haarkur

| | |
|---|---|
| Sepigel^{®}305 | 5,0 |
| Dow Corning^{®}Q2-5220¹⁶ | 1,5 |
| Ajidew^{®} NL 50 | 3,0 |
| Polymer P1 entsprechend DE 3929173 | 0,6 |
| Genamin^{®}DSAC¹⁷ | 0,3 |
| Phenonip^{®} | 0,8 |
| Parfümöl | 0,25 |
| Wasser | ad 100 |

| | |
|---|---|
| ¹⁶ Silicon-Glykol-Copolymer (INCI-Bezeichnung: Dimethicone Copolyol) (DOW CORNING) ¹⁷ Dimethyldistearylammoniumchlorid (INCI-Bezeichnung: Distearyldimonium Chloride) (CLARIANT) | |

### 7. Shampoo

| | |
|---|---|
| Texapon^{®} NSO¹⁸ | 40,0 |
| Dehyton^{®} G¹⁹ | 6,0 |
| Polymer JR 400^{®20} | 0,5 |
| Ajidew^{®} NL 50 | 2,5 |
| Cetiol^{®} HE²¹ | 0,5 |
| Citronensäure | 0,5 |
| Natriumbenzoat | 0,5 |
| Parfüm | 0,4 |
| NaCl | 0,5 |
| Wasser | ad 100 |

| | |
|---|---|
| ¹⁸ Natriumlaurylethersulfat ca. 28% Aktivsubstanz (INCI - Bezeichnung: Sodium Laureth Sulfate) (COGNIS) ¹⁹ INCI - Bezeichnung: Sodium Cocoamphoacetate ca. 30% Aktivsubstanz (COGNIS) ²⁰ quaternierte Hydroxyethylcellulose (INCI - Bezeichnung: Polyquaternium-10) (UNION CARBIDE) ²¹ Polyol-Fettsäure-Ester (INCI - Bezeichnung: PEG-7 Glyceryl Cocoate) (COGNIS) | |

### 8. Shampoo

| | |
|---|---|
| Texapon^{®} NSO | 43,0 |
| Dehyton^{®} K²² | 10,0 |
| Plantacare^{®} 1200 UP²³ | 4,0 |
| Euperlan^{®}PK 3000²⁴ | 1,6 |
| Arquad^{®}316²⁵ | 0,8 |
| Polymer JR^{®} 400 | 0,3 |
| Ajidew^{®}NL 50 | 4,0 |
| Glucamate^{®}DOE 120²⁶ | 0,5 |
| Natriumchlorid | 0,2 |
| Wasser | ad 100 |

| | |
|---|---|
| ²² INCI - Bezeichnung: Cocamidopropyl Betaine ca. 30% Aktivsubstanz (COGNIS) ²³ C 12 - C 16 Fettalkoholglycosid ca. 50% Aktivsubstanz (INCI - Bezeichnung: Lauryl Glucoside) (COGNIS) ²⁴ Flüssige Dispersion von perlglanzgebenden Substanzen und Amphotensid (ca. 62 % Aktivsubstanz; CTFA-Bezeichnung: Glycol Distearate (and) Glycerin (and) Laureth-4 (and) Cocoamidopropyl Betaine) (COGNIS) ²⁵ Tri-C₁₆-alkylmethylammoniumchlorid (AKZO) ²⁶ ethoxyliertes Methylglucosid-dioleat (CTFA-Bezeichnung: PEG-120 Methyl Glucose Dioleate) (AMERCHOL) | |

### 9. Shampoo

| | |
|---|---|
| Texapon^{®}N 70²⁷ | 21,0 |
| Plantacare^{®} 1200 UP | 8,0 |
| Gluadin^{®} WQ²⁸ | 1,5 |
| Cutina^{®} EGMS²⁹ | 0,6 |
| Honeyquat^{®} 50 | 2,0 |
| DL-2-Pyrrolidinon-5-carbonsäure (Fa. Merck) | 2,0 |
| Antil^{®} 141³⁰ | 1,3 |
| Natriumchlorid | 0,2 |
| Magnesiumhydroxid | ad pH 4,5 |
| Wasser | ad 100 |

| | |
|---|---|
| ²⁷ Natriumlaurylethersulfat mit 2 Mol EO ca. 70% Aktivsubstanz (INCI - Bezeichnung: Sodium Laureth Sulfate) (COGNIS) ²⁸ kationisiertes Weizenproteinhydrolysat ca. 31% Aktivsubstanz (INCI - Bezeichnung: Laurdimonium Hydroxypropyl Hydrolyzed Wheat Protein) (COGNIS) ²⁹ Ethylenglykolmonostearat (ca. 25-35% Monoester, 60-70% Diester; INCI - Bezeichnung: Glycol Stearate) (COGNIS) ³⁰ Polyoxyethylen-propylenglykoldioleat (40 % Aktivsubstanz; INCI - Bezeichnung: Propylene Glycol (and) PEG-55 Propylene Glycol Oleate) (GOLDSCHMIDT) | |

### 10. Shampoo

| | |
|---|---|
| Texapon^{®} K 14 S³¹ | 50,0 |
| Dehyton^{®} K | 10,0 |
| Planlacare^{®} 818 UP³² | 4,5 |
| Polymer P1, entsprechend DE 39 29 973 | 0,6 |
| Cutina^{®} AGS³³ | 2.0 |
| D-Panthenol | 0,5 |
| Glucose | 1,0 |
| Salicylsäure | 0,4 |
| Natriumchlorid | 0,5 |
| Ajidew^{®} NL 50 | 2,0 |
| Wasser | ad 100 |

| | |
|---|---|
| ³¹ Natriumlaurylmyristylethersulfat ca 28% Aktivsubstanz (INCI - Bezeichnung: Sodium Myreth Sulfate) (COGNIS) ³² C 8 - C 16 Fettalkoholglycosid ca. 50% Aktivsubstanz (INCI - Bezeichnung: Coco Glucoside) (COGNIS) ³³ Ethylenglykolstearat (ca. 5-15% Monoester, 85-95% Diester; INCI - Bezeichnung: Glycol Distearate) (COGNIS) | |

### 11. Haarkur

| | |
|---|---|
| Celquat^{®} L 200³⁴ | 0,6 |
| Luviskol^{®} K30³⁵ | 0,2 |
| D-Panthenol | 0,5 |
| Polymer P1, entsprechend DE 39 29 973 | 0,6 |
| Dehyquart^{®} A-CA³⁶ | 1,0 |
| Gluadin^{®} W 40³⁷ | 1,0 |
| Natrosol^{®} 250 HR³⁸ | 1,1 |
| Ajidew^{®} NL 50 | 2,0 |
| Wasser | ad 100 |

| | |
|---|---|
| ³⁴ quaterniertes Cellulose-Derivat (95 % Aktivsubstanz; CTFA-Bezeichnung: Polyquaternium-4) (DELFT NATIONAL) ³⁵ Polyvinylpyrrolidon (95 % Aktivsubstanz; CTFA-Bezeichnung: PVP) (BASF) ³⁶ Cetyltrimethylammoniumchlorid (INCI - Bezeichnung: Cetrimonium Chloride) (COGNIS) ³⁷ Partialhydrolysat aus Weizen ca. 40% Aktivsubstanz (INCI - Bezeichnung: Hydrolyzed Wheat Gluten Hydrolyzed Wheat Protein) (COGNIS) ³⁸ Hydroxyethylcellulose (AQUALON) | |

### 12. Färbecreme

| | |
|---|---|
| C₁₂₋₁₈-Fettalkohol | 1,2 |
| Lanette^{®} O³⁹ | 4,0 |
| Eumulgin^{®} B 2 | 0,8 |
| Cutina^{®} KD 16⁴⁰ | 2,0 |
| Natriumsulfit | 0,5 |
| L(+)-Ascorbinsäure | 0,5 |
| Ammoniumsulfat | 0,5 |
| 1,2-Propylenglykol | 1,2 |
| Polymer JR^{®}400 | 0,3 |
| p-Aminophenol | 0,35 |
| p-Toluylendiamin | 0,85 |
| 2-Methylresorcin | 0,14 |
| 6-Methyl-m-aminophenol | 0,42 |
| Cetiol^{®} OE⁴¹ | 0,5 |
| Honeyquat^{®} 50 | 1,0 |
| (R)-(+)-2-Pyrrolidinon-5-carbonsäure | 1,0 |
| Gluadin^{®} W 40 | 1,0 |
| Ammoniak | 1,5 |
| Wasser | ad 100 |

| | |
|---|---|
| ³⁹ Cetylstearylalkohol (INCI - Bezeichnung: Cetearyl Alcohol) (COGNIS) ⁴⁰ Selbstemulgierendes Gemisch aus Mono- / Diglyceriden höherer gesättigter Fettsäuren mit Kaliumstearat (INCI - Bezeichnung: Glyceryl Stearate SE) (COGNIS) ⁴¹ Di-n-octylether (INCI - Bezeichnung: Dicaprylyl Ether) (COGNIS) | |

### 13. Entwicklerdispersion für Färbecreme 12.

| | |
|---|---|
| Texapon^{®} NSO | 2.1 |
| Wasserstoffperoxid (50%ig) | 12,0 |
| Turpinal^{®} SL⁴² | 1.7 |
| Latekoll^{®} D⁴³ | 12,0 |
| Ajidew^{®} NL 50 | 0,3 |
| Salcare^{®} SC 96 | 1,0 |
| Wasser | ad 100 |

| | |
|---|---|
| ⁴² 1-Hydroxyethan-1,1-diphosphonsäure (60 % Aktivsubstanz; INCI - Bezeichnung: Etidronic Acid) (COGNIS) ⁴³ Acrylester-Methacrylsäure-Copolymer (25 % Aktivsubstanz) (BASF) | |

Die Färbecreme hatte einen pH-Wert von 10,0. Sie bewirkte eine intensive rote Tönung des Haares.

### 14. Tönungsshampoo

| | |
|---|---|
| Texapon^{®} N 70 | 14,0 |
| Dehyton^{®} K | 10,0 |
| Akypo^{®} RLM 45 NV⁴⁴ | 14,7 |
| Plantacare^{®} 1200 UP | 4,0 |
| Polymer P1, entsprechend DE 39 29 973 | 0,3 |
| Cremophor^{®} RH 40⁴⁵ | 0,8 |
| Farbstoff C.I. 12 719 | 0,02 |
| Farbstoff C.I. 12 251 | 0,02 |
| Farbstoff C.I. 12 250 | 0,04 |
| Farbstoff C.I. 56 059 | 0,03 |
| Konservierung | 0,25 |
| Parfümöl | q.s. |
| Eutanol^{®} G⁴⁶ | 0,3 |
| Ajidew^{®} NL 50 | 1,0 |
| Honeyquat^{®} 50 | 1,0 |
| Salcare^{®} SC 96 | 0,5 |
| Wasser | ad 100 |

| | |
|---|---|
| ⁴⁴ Laurylalkohol+4.5 Ethylenoxid-essigsäure-Natriumsalz (20,4 % Aktivsubstanz) (CHEM-Y) ⁴⁵ Rizinus-Öl, hydriert + 45 Ethylenoxid (INCI - Bezeichnung: PEG-40 Hydrogenated Castor Oil) (BASF) ⁴⁶ 2-Octyldodecanol (Guerbet-Alkohol) (INCI - Bezeichnung: Octyldodecanol) (COGNIS) | |

Beim Waschen der Haare mit diesem Tönungs-Shampoo erhalten diese einen glänzenden, hellblonden Farbton.

### 15. Cremedauerwelle

### Wellcreme

| | |
|---|---|
| Plantacare^{®} 810 UP⁴⁶ | 5,0 |
| Thioglykolsäure | 8,0 |
| Turpinal^{®} SL | 0,5 |
| Ammoniak (25%ig) | 7,3 |
| Ammoniumcarbonat | 3,0 |
| Cetyl/Stearyl-Alkohol | 5,0 |
| Guerbet-Alkohol | 4,0 |
| Salcare^{®} SC 96 | 3,0 |
| Ajidew^{®} NL 50 | 2,0 |
| Parfümöl | q.s. |
| Wasser | ad 100 |

| | |
|---|---|
| ⁴⁷ C₈-C₁₀-Alkylglucosid mit Oligomerisationsgrad 1,6 (ca. 60% Aktivsubstanz) (COGNIS) | |

### Fixierlösung

| | |
|---|---|
| Plantacare^{®} 810 UP | 5,0 |
| gehärtetes Rizinusöl | 2,0 |
| Kaliumbromat | 3,5 |
| Nitrilotriessigsäure | 0,3 |
| Zitronensäure | 0,2 |
| Merquat^{®} 550⁴⁷ | 0,5 |
| Hydagen^{®} HCMF⁴⁸ | 0,5 |
| Ajidew^{®} NL 50 | 0,5 |
| Parfümöl | q.s. |
| Wasser | ad 100 |

| | |
|---|---|
| ⁴⁸ Dimethyldiallylammoniumchlorid-Acrylamid-Copolymer (8 % Aktivsubstanz; INCI - Bezeichnung: Polyquarternium 7) (MOBIL OIL) ⁴⁹ Chitosan Pulver (INCI - Bezeichnung: Chitosan) (COGNIS) | |

## Patentansprüche

1. Verwendung eines Wirkstoffkomplexes bestehend aus
- einer Pyrrolidinoncarbonsäure und/oder eines Pyrrolidinoncarbonsäurederivates (A) der Formel (I), in der mindestens einer der Substituenten R¹ bis R³ für eine Gruppe -COOR⁴ steht, in der R⁴ Wasserstoff, ein Alkalimetallion, ein Erdalkalimetallion oder ein Ammoniumion ⁺NHR⁵R⁶R⁷ ist, in dem R⁵ bis R⁷ unabhängig voneinander Wasserstoff, Alkylgruppen mit 1 bis 22 C-Atomen, Hydroxyalkylgruppen mit 1 bis 4 C-Atomen, Alkenylgruppen mit 2 bis 22 C-Atomen, Acylgruppen mit 2 bis 22 C-Atomen oder gegebenenfalls substituierte aromatische Gruppen mit 6 bis 10 C-Atomen sind, und die restlichen Substituenten R¹ bis R³ für Wasserstoff oder Alkylgruppen mit 1 bis 4 C-Atomen stehen, 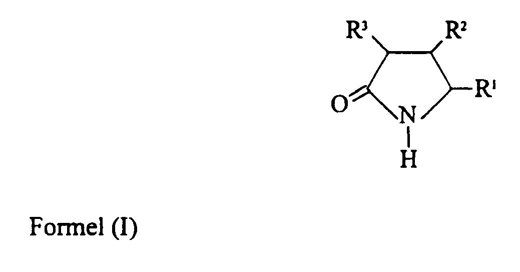
- und eines natürlichen oder synthetischen kationischen oder amphoteren Polymeren (B) zur Verbesserung der Waschechtheit von Färbungen von Fasern, insbesondere von keratinischen Fasern.

2. Verwendung nach Anspruch 1, **dadurch gekennzeichnet, daß** die Verbindung (A) der allgemeinen Formel (I) 2-Pyrrolidinon-5-carbonsäure oder ein Salz dieser Säure ist.

3. Verwendung nach Anspruch 2, **dadurch gekennzeichnet, daß** die Verbindung (A) der allgemeinen Formel (I) das Natriumsalz der 2-Pyrrolidinon-5-carbonsäure ist.

4. Verwendung nach einem der Ansprüche 1 bis 3, **dadurch gekennzeichnet, daß** das Polymer ein natürliches oder synthetisches kationisches Polymer ist.

5. Verwendung nach Anspruch 4, **dadurch gekennzeichnet, daß** das kationische Polymer ausgewählt ist aus Homopolymeren der allgemeinen Formel (II), 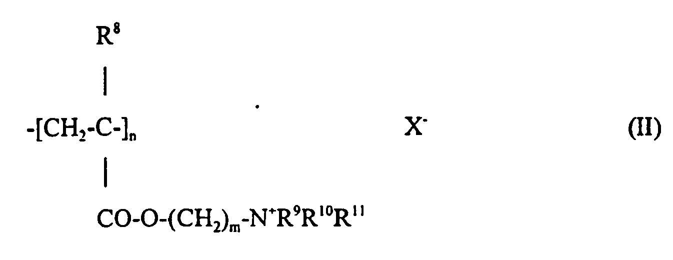 in der R⁸ = -H oder -CH₃ ist, R⁹, R¹⁰ und R¹¹ unabhängig voneinander ausgewählt sind aus C₁₋₄-Alkyl-, -Alkenyl- oder -Hydroxyalkylgruppen, m = 1, 2, 3 oder 4, n eine natürliche Zahl und X⁻ ein physiologisch verträgliches organisches oder anorganisches Anion ist, und Copolymeren, bestehend im wesentlichen aus den in Formel (II) aufgeführten Monomereinheiten sowie nichtionogenen Monomereinheiten.

6. Verwendung nach Anspruch 4, **dadurch** ausgewählt, daß das Polymer (B) Chitosan oder ein Derivat des Chitosanes ist.

7. Verwendung nach Anspruch 4, **dadurch gekennzeichnet, daß** die kationischen Polymere (B) ausgewählt sind aus der Gruppe, die
- kationisierte Cellulose-Derivate,
- kationisierte Guar-Derivate,
- kationisierten Honig und/oder
- kationische Alkylpolyglycoside umfaßt.

8. Verwendung nach einem der Ansprüche 1 bis 3, **dadurch gekennzeichnet, daß** das Polymer (B) ein amphoteres Polymer ist.

9. Verwendung nach einem der Ansprüche 1 bis 8, **dadurch gekennzeichnet, daß** der Wirkstoffkomplex in Kombination mit einem Proteinhydrolysat oder einem Derivat eines Proteinhydrolysats eingesetzt wird.

10. Verwendung nach Anspruch 9, **dadurch gekennzeichnet, daß** das Proteinhydrolysat oder Derivat des Proteinhydrolysats pflanzlichen Ursprungs ist.

11. Verwendung nach einem der Ansprüche 1 bis 10, **dadurch gekennzeichnet, daß** der Wirkstoffkomplex in Kombination mit einem kationischen Tensid eingesetzt wird.

12. Verwendung nach einem der Ansprüche 1 bis 11, **dadurch gekennzeichnet, daß** der Wirkstoffkomplex in Kombination mit einem Vitamin, einem Provitamin, einer Vitaminvorstufe oder einem Derivat davon eingesetzt wird.

13. Verwendung nach einem der Ansprüche 1 bis 12, **dadurch gekennzeichnet, daß** der Wirkstoffkomplex in Kombination mit einem Pflanzenextrakt eingesetzt wird.

14. Verfahren zur Färbung von Fasern, insbesondere keratinischer Fasern, **dadurch gekennzeichnet, daß** in einem ersten Schritt die Fasern in üblicher Weise gefärbt werden und in einem zweiten Schritt ein Mittel auf die Fasern aufgetragen wird, das mindestens den Wirkstoffkomplex gemäß einem der Ansprüche 1 bis 13 zur Erhöhung der Waschechtheit der Färbung enthält, wobei das Mittel gewünschtenfalls nach einer Einwirkzeit von 1 bis 5 Minuten wieder ausgespült wird.

15. Verfahren zur Färbung von Fasern, insbesondere keratinischer Fasern, **dadurch gekennzeichnet, daß** in einem ersten Schritt ein Mittel auf die Fasern aufgetragen wird, das mindestens den Wirkstoffkomplex gemäß einem der Ansprüche 1 bis 13 zur Erhöhung der Waschechtheit der Färbung enthält, und anschließend in einem zweiten Schritt die keratinischen Fasern in üblicher Weise gefärbt werden.

## Claims

1. Use of an active ingredient complex consisting of
- a pyrrolidinonecarboxylic acid and/or a pyrrolidinonecarboxylic acid derivative (A) of the formula (I), in which at least one of the substituents R¹ to R³ is a group -COOR⁴, in which R⁴ is hydrogen, an alkali metal ion, an alkaline earth metal ion or an ammonium ion ⁺NHR⁵R⁶R⁷, in which R⁵ to R⁷, independently of one another, are hydrogen, alkyl groups having 1 to 22 carbon atoms, hydroxyalkyl groups having 1 to 4 carbon atoms, alkenyl groups having 2 to 22 carbon atoms, acyl groups having 2 to 22 carbon atoms or optionally substituted aromatic groups having 6 to 10 carbon atoms, and the remaining substituents R¹ to R³ are hydrogen or alkyl groups having 1 to 4 carbon atoms, 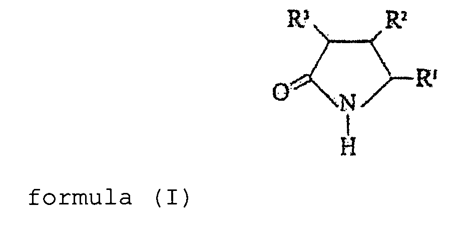
- and a natural or synthetic cationic or amphoteric polymer (B) for improving the washfastness of colourations of fibres, in particular of keratin fibres.

2. Use according to Claim 1, **characterized in that** the compound (A) of the general formula (I) is 2-pyrrolidinone-5-carboxylic acid or a salt of this acid.

3. Use according to Claim 2, **characterized in that** the compound (A) of the general formula (I) is the sodium salt of 2-pyrrolidinone-5-carboxylic acid.

4. Use according to one of Claims 1 to 3, **characterized in that** the polymer is a natural or synthetic cationic polymer.

5. Use according to Claim 4, **characterized in that** the cationic polymer is selected from homopolymers of the general formula (II), 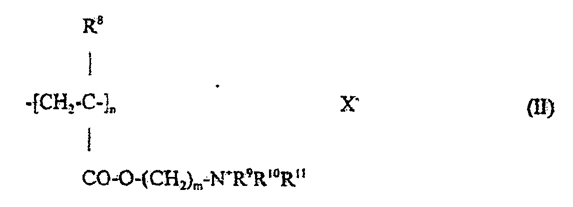 in which R⁸ = -H or -CH₃, R⁹, R¹⁰ and R¹¹, independently of one another, are selected from C₁₋₄-alkyl, -alkenyl or -hydroxyalkyl groups, m = 1, 2, 3 or 4, n is a natural number and X⁻ is a physiologically compatible organic or inorganic anion, and copolymers consisting essentially of the monomer units given in formula (II), and also nonionogenic monomer units.

6. Use according to Claim 4, **characterized in that** the polymer (B) is chitosan or a derivative of chitosan.

7. Use according to Claim 4, **characterized in that** the cationic polymers (B) are selected from the group which includes
- cationized cellulose derivatives,
- cationized guar derivatives,
- cationized honey and/or
- cationic alkyl polyglycosides.

8. Use according to one of Claims 1 to 3, **characterized in that** the polymer (B) is an amphoteric polymer.

9. Use according to one of Claims 1 to 8, **characterized in that** the active ingredient complex is used in combination with a protein hydrolysate or a derivative of a protein hydrolysate.

10. Use according to Claim 9, **characterized in that** the protein hydrolysate or derivative of the protein hydrolysate is of vegetable origin.

11. Use according to one of Claims 1 to 10, **characterized in that** the active ingredient complex is used in combination with a cationic surfactant.

12. Use according to one of Claims 1 to 11, **characterized in that** the active ingredient complex is used in combination with a vitamin, a provitamin, a vitamin precursor or a derivative thereof.

13. Use according to one of Claims 1 to 12, **characterized in that** the active ingredient complex is used in combination with a plant extract.

14. Method of colouring fibres, in particular keratin fibres, **characterized in that**, in a first step, the fibres are coloured in the customary manner and, in a second step, a composition is applied to the fibres which comprises at least the active ingredient complex according to one of Claims 1 to 13 for increasing the washfastness of the colouration, where the composition is, if desired, rinsed out again after a contact time of from 1 to 5 minutes.

15. Method of colouring fibres, in particular keratin fibres, **characterized in that**, in a first step, a composition is applied to the fibres which comprises at least the active ingredient complex according to one of Claims 1 to 13 for increasing the washfastness of the colouration, and then, in a second step, the keratin fibres are coloured in the customary manner.

## Revendications

1. Utilisation d'un complexe de substance active constitué
- d'un acide pyrrolidinone-carboxylique et/ou d'un dérivé d'acide pyrrolidinone-carboxylique (A) de formule (1), dans laquelle au moins l'un des substituants R¹ à R³ représente un groupe -COOR⁴, dans lequel R⁴ représente un atome d'hydrogène, un ion de métal alcalin, un ion de métal alcalino-terreux ou un ion ammonium ⁺NHR⁵R⁶R⁷, dans lequel R⁵ à R⁷ représentent indépendamment l'un de l'autre un atome d'hydrogène, des groupes alkyle ayant de 1 à 22 atomes de C, des groupes hydroxyalkyle ayant de 1 à 4 atomes de C, des groupes alcényle ayant de 2 à 22 atomes de C, des groupes acyle ayant de 2 à 22 atomes de C ou des groupes aromatiques éventuellement substitués ayant de 6 à 10 atomes de C, et les substituants restants R¹ à R³ représentent un atome d'hydrogène ou des groupes alkyle ayant de 1 à 4 atomes de C, 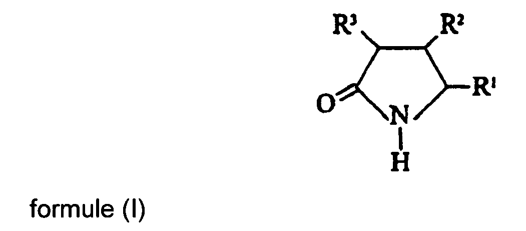
- et d'un polymère cationique ou amphotère, naturel ou synthétique (B) pour améliorer la résistance au lavage des colorations de fibres, en particulier de fibres kératiniques.

2. Utilisation selon la revendication 1, **caractérisée en ce que** le composé (A) de formule générale (I) représente l'acide 2-pyrrolidinone-5-carboxylique ou un sel de cet acide.

3. Utilisation selon la revendication 2, **caractérisée en ce que** le composé (A) de formule générale (I) est le sel sodique de l'acide 2-pyrrolidinone-5-carboxylique.

4. Utilisation selon l'une quelconque des revendications 1 à 3, **caractérisée en ce que** le polymère est un polymère cationique naturel ou synthétique.

5. Utilisation selon la revendication 4, **caractérisée en ce que** le polymère cationique est choisi parmi les homopolymères de formule générale (II), 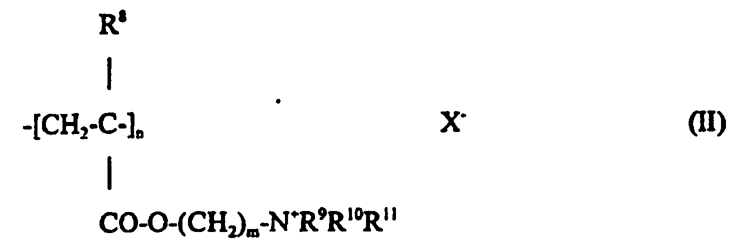 dans laquelle R⁸ = -H ou -CH₃, R⁹, R¹⁰ et R¹¹ sont choisis indépendamment l'un de l'autre parmi des groupes alkyle, alcényle ou hydroxyalkyle en Ci à C₄, m = 1,2,3 ou 4, n est un nombre naturel et X⁻ est un anion organique ou inorganique, acceptable sur le plan physiologique, et les copolymères constitués essentiellement des unités monomères énumérées dans la formule (11) ainsi que des unités monomères non ionogènes.

6. Utilisation selon la revendication 4, **caractérisée en ce que** le polymère (B) est le chitosan ou un dérivé du chitosan.

7. Utilisation selon la revendication 4, **caractérisée en ce que** les polymères cationiques (B) sont choisis dans le groupe qui comprend
- les dérivés de cellulose cationisés,
- les dérivés de guar cationisés,
- le miel cationisé et/ou
- les alkylpolyglycosides cationiques.

8. Utilisation selon l'une quelconque des revendications 1 à 3, **caractérisée en ce que** le polymère (B) est un polymère amphotère.

9. Utilisation selon l'une quelconque des revendications 1 à 8, **caractérisée en ce que** le complexe de substance active est mis en oeuvre en combinaison avec un hydrolysat de protéine ou un dérivé d'un hydrolysat de protéine.

10. Utilisation selon la revendication 9, **caractérisée en ce que** l'hydrolysat de protéine ou le dérivé de l'hydrolysat de protéine est d'origine végétale.

11. Utilisation selon l'une quelconque des revendications 1 à 10, **caractérisée en ce que** le complexe de substance active est mis en oeuvre en combinaison avec un tensio-actif cationique.

12. Utilisation selon l'une quelconque des revendications 1 à 11, **caractérisée en ce que** le complexe de substance active est mis en oeuvre en combinaison avec une vitamine, une provitamine, un précurseur de vitamine ou un dérivé de ceux-ci.

13. Utilisation selon l'une quelconque des revendications 1 à 12, **caractérisée en ce que** le complexe de substance active est mis en oeuvre en combinaison avec un extrait végétal.

14. Procédé pour teindre les fibres, en particulier les fibres kératiniques, **caractérisé en ce que** dans une première étape, les fibres sont teintes d'une façon habituelle, et dans une deuxième étape, un agent est appliqué sur les fibres, qui contient au moins le complexe de substance active selon l'une quelconque des revendications 1 à 13 pour augmenter la résistance au lavage de la coloration, l'agent étant éliminé de nouveau si on le souhaite par rinçage au bout d'une durée d'action de 1 à 5 minutes.

15. Procédé pour teindre les fibres, en particulier les fibres kératiniques, **caractérisé en ce que** dans une première étape, un agent est appliqué sur les fibres, qui contient au moins le complexe de substance active selon l'une quelconque des revendications 1 à 13 pour augmenter la résistance au lavage de la coloration, et ensuite dans une deuxième étape, les fibres kératiniques sont teintes d'une façon habituelle.
